(19) Europäisches Patentamt
European Patent Office
Office européen des brevets

(11) **EP 1 711 832 B1**

(12) **EUROPEAN PATENT SPECIFICATION**

(45) Date of publication and mention
of the grant of the patent:
**06.08.2008 Bulletin 2008/32**

(51) Int Cl.:
**G01N 33/68** (2006.01)

(21) Application number: **05706885.0**

(22) Date of filing: **14.01.2005**

(86) International application number:
**PCT/EP2005/000301**

(87) International publication number:
**WO 2005/069008 (28.07.2005 Gazette 2005/30)**

(54) **METHOD FOR EXAMINING THE ACTIVITY OF ION CHANNELS**

VERFAHREN ZUR UNTERSUCHUNG DER AKTIVITÄT VON IONENKANÄLEN

PROCEDE POUR ETUDIER L'ACTIVITE DE CANAUX IONIQUES

(84) Designated Contracting States:
**AT BE BG CH CY CZ DE DK EE ES FI FR GB GR HU IE IS IT LI LT LU MC NL PL PT RO SE SI SK TR**

(30) Priority: **15.01.2004 US 536514 P**

(43) Date of publication of application:
**18.10.2006 Bulletin 2006/42**

(73) Proprietor: **Evotec AG**
**22525 Hamburg (DE)**

(72) Inventors:
• **EBNETH, Andreas**
**25421 Pinneberg (DE)**
• **NETZER, Rainer**
**22609 Hamburg (DE)**
• **HAHN, Ulrike**
**21391 Reppenstedt (DE)**

(74) Representative: **Meyers, Hans-Wilhelm**
**Patentanwälte**
**von Kreisler-Selting-Werner**
**Postfach 10 22 41**
**50462 Köln (DE)**

(56) References cited:
**WO-A-02/31508**

• **SIEM-FUNG D J ET AL: "THE EFFECT OF TEMPERATURE ON VERATRIDINE ACTION IN SQUID GIANT AXONS" BIOCHIMICA ET BIOPHYSICA ACTA, vol. 728, no. 3, 1983, pages 305-310, XP008045560 ISSN: 0006-3002**

• **KIM C S ET AL: "Voltage-dependent calcium channels in ventricular cells of rainbow trout: effect of temperature changes in vitro." AMERICAN JOURNAL OF PHYSIOLOGY. REGULATORY, INTEGRATIVE AND COMPARATIVE PHYSIOLOGY. JUN 2000, vol. 278, no. 6, June 2000 (2000-06), pages R1524-R1534, XP002324413 ISSN: 0363-6119**

• **MITSUIYE TAMOTSU ET AL: "Temperature dependence of the inward rectifier K+ channel gating in guinea-pig ventricular cells" JAPANESE JOURNAL OF PHYSIOLOGY, vol. 47, no. 1, 1997, pages 73-79, XP002324414 ISSN: 0021-521X**

• **CHUNG SHIN-HO ET AL: "Changes in the kinetics and conductance of N-methyl-D-aspartate (NMDA)-receptor activated single channels with temperature" NEUROSCIENCE LETTERS, vol. 187, no. 3, 1995, pages 181-184, XP002324415 ISSN: 0304-3940**

• **DING J P ET AL: "Modulation of mechanosensitive calcium-selective cation channels by temperature." THE PLANT JOURNAL : FOR CELL AND MOLECULAR BIOLOGY. MAY 1993, vol. 3, no. 5, May 1993 (1993-05), pages 713-720, XP002324416 ISSN: 0960-7412**

• **NETZER RAINER ET AL: "Screening lead compounds for QT interval prolongation" DRUG DISCOVERY TODAY, ELSEVIER SCIENCE LTD, GB, vol. 6, no. 2, January 2001 (2001-01), pages 78-84, XP002198162 ISSN: 1359-6446**

• **NUMANN R ET AL: "High-throughput screening strategies for cardiac ion channels" TRENDS IN CARDIOVASCULAR MEDICINE 2001 UNITED STATES, vol. 11, no. 2, 2001, pages 54-59, XP002324417 ISSN: 1050-1738**

**Description**

The present invention relates to a method for examining the activity of ion channels.

The membranes of living cells serve a wide variety of functions which are of great importance to the integrity and activity of cells and tissues. The delimitation and regulation of the cell contents, exchange of matter and transmission of signals are examples of such functions. Charged molecules and inorganic ions (such as $Na^+$, $K^+$, $Ca^{2+}$ and $Cl^-$ ions) cannot cross membranes by simple diffusion through the lipid bilayer, but require specific transport systems of the membrane. In particular, such transport systems comprise ion channels, of which a wide variety are very well characterized, inter alia, in terms of their biochemical and electrophysiological properties, not least because of their immense importance to various clinical pictures. Such ion channels can be opened and closed selectively, so that ions cannot constantly flow through. The net flow of individual ions is determined by factors like the permeability for the respective ions, the concentration gradient of the ion and the electric potential difference between the two sides of the membrane. Generally, ion channel types which respond to a change of electric potential (voltage-dependent ion channels) are distinguished from those which respond to specific messengers, so-called transmitters. Further, ion pumps are known which provide for active ion transport against the electrochemical gradient with consumption of energy. In this way, characteristic differences, in the ion concentrations between the intracellular and extracellular spaces are generated or maintained. One important example is the so-called sodium-potassium pump which enables a coupled transport of sodium and potassium with consumption of ATP as energy source.

A wide variety of clinical pictures are known which are treated with drugs by selectively influencing the activity of ion channels. These include, inter alia, anti-arrhythmic agents, i.e., agents for the treatment of cardiac arrhythmia, which are subdivided into different classes according to their electrophysiological mechanisms of action. Thus, for example, the calcium antagonist verapamil acts through the blocking of calcium channels, whereas members of the potassium antagonists, such as amiodarone and sotalol, cause a selective extension of the duration of action potentials by blocking potassium channels. Siem-Fung andSevcik (Biochimica et Biophysica Acta, 728, 305-310, 1983) analysed the action of veratridine in squid giant axons at different temperatures. Other clinical pictures which can be influenced by activating or blocking ion channels include, for example, a large number of CNS diseases (e. g. epilepsy, pain, stroke, migraine), auto-immune diseases, cancer or diabetes.

Within the scope of research on pharmaceutically active substances, it is desirable to have test methods by which the influence of a potentially pharmacologically active substance on such ion channels can be exactly monitored. This is of importance, on the one hand, in the development of substances whose mechanism of action is based on a selective affection of ion channels, and on the other hand, in the evaluation of potential side effects, i.e., the undesirable affection of ion channel activities by the putative drugs.

The membrane potential of living cells is predominantly determined by the intracellular and extracellular sodium, potassium and chloride ion concentrations. For example, if one examines the influence of the blocking of a potassium channel on the resting membrane potential of living cells, the conductivity for potassium through the membrane is essentially changed according to the Goldmann-Hodgkin-Katz equation, which has an effect on the membrane potential. Cells can respond to this change, inter alia, by changing the conductivity for other ions through the membrane to reduce or even prevent a net influence of the potassium channel blocking on the membrane potential. This can be done through the activation of pump systems in the cells which actively transport ions.

Now, if one monitors the membrane potential of living cells under the influence of a potential or known pharmacologically active substance in a test method, there is a risk that the potential value measured is biased due to the counter-regulation mechanisms described. This biasing can even be so high that an affection of the membrane potential may not be recognizable when the signal-to-noise ratio is unfavorably high.

Thus, it is the object of the present invention to provide a test method for examining the activity of ion channels which minimizes the above mentioned interferences.

This object is achieved by a method having the features of independent claim 1. The further claims dependent on claim 1 relate to preferred embodiments of the present invention. Additional claims are directed to the use of specific means to conduct the method according to the present invention.

The present invention relates to a method according to claim 1.

The present invention is based on the recognition that, by decreasing the temperature, it is possible to deprive the cells of the possibility of influencing the membrane potential by the activation of the above mentioned pump systems. Test methods described in the prior art, e.g., for the evaluation of potassium channel blockers, are typically performed at body temperature, i.e., 37 °C, or at room temperature. If the cells are deprived of the possibility of influencing the membrane potential by the activation of the above mentioned pump systems (or if such possibility is at least significantly reduced) by reducing the temperature, the cells cannot respond as effectively to a change of the membrane potential by blocking potassium or sodium channels as they can at 37 °C or at room temperature.

According to the present invention, it is preferred to perform a determination of the measuring parameter at temperatures of $\leq 10$ °C, especially at $\leq 5$ °C. Particularly preferred are temperatures of $\leq 2$°C. The lower temperature limit is preferably 0 °C. Since the samples (e. g. cell samples) to be examined are typically contained in isotonic buffer solutions, it is also possible, in principle, to perform the measurement slightly below 0 °C, typically down to -2 °C or -4 °C.

Typically, the measurements are conducted utilizing cells which contain the ion channels. Such cells might be wild-type cells or might be genetically modified cells which e. g. over-express the ion channel under study. However, it is also possible to conduct the measurements according to the present invention on tissues or on cell organelles such as mitochondria. In a further embodiment, it is also possible to prepare membrane fractions or vesicles containing the ion channels of interest and to conduct the measurements according to the present invention on such preparations. Membrane fractions and vesicles might be prepared according to standard methods known in the art of cell fractionation, typically by a lysis of cell pellets obtained from centrifugation of cells in buffer comprising protease inhibitors. The lysate is then typically centrifuged again to pellet debris and organelles. The resulting supernatant typically is spun to collect membranes. Afterwards, the resultant pellet is re-suspended in appropriate buffer to conduct the measurements according to the present invention. If desired, vesicles with uniform size can be obtained through brief sonication. As a further alternative, one might conduct the measurements according to the present invention on ion channels embedded into artificial membranes.

Ion channels to be studied according to the present invention are typically associated with membranes, such as the plasma membrane of cells, the membrane of cell organelles, vesicular membrane or even an artificial membrane. Typically, human or animal cells and cell organelles are used as such, or vesicles or membrane fractions are prepared from such cells and cell organelles. As taught by the present invention, such ion channels are examined by determining a value of a measuring parameter as an Indicator of the activity of the ion channels at a decreased temperature compared to room temperature or body temperature. Preferably, the measuring parameter is the membrane potential of the cell, cell organelle, vesicle or artificial membrane, or a measure thereof. In a further embodiment, the measuring parameter might be an ion concentration or a measure thereof. The concentrations of ions such as potassium, sodium, chloride and/or calcium might be studied. Preferably, the measuring parameter is an extracellular and/or intracellular ion concentration of the ions mentioned above, or a measure thereof.

The value of the measuring parameter is established before; during and/or after the addition of a test substance which (potentially) influences the activity of the ion channels under study. In particular, the activity of a transmitter-dependant ion channel can be examined. However, it is also possible to establish the activity of a voltage-sensitive ion channel. This may be, in particular, the activity of a potassium channel, sodium channel, chloride channel or calcium channel.

Said establishing of a measure of the ion concentration or the membrane potential is effected by fluorescence methods.

Many different means may be used to measure the membrane potential or the ion concentration, including but not limited to ion-sensitive or voltage-sensitive dyes and chelating agents.

For example, the fluorescence emission of a voltage-sensitive fluorescent dye, especially the dye $Dibac_4(3)$, can serve as a measure of the membrane potential, as set forth in more detail below. Further, the ion concentration of calcium, for example, may be measured by means of chelating agents.

The determination of the membrane potential, which is to be performed at the low temperatures according to the invention, may be effected, in particular, by means of per se known fluorescence assays using commercially available fluorescence readers (e.g., FLIPR of Molecular Devices), confocal fluorescence microscopes or flow-cytometric apparatus. Typically, potential-sensitive fluorescence dyes, such as the commercially available distribution dye bis (1,3-dibutylbarbituric acid)trimethine oxonol ($Dibac_4(3)$), can be employed. $Dibac_4(3)$ is a dye of the bisoxonol type whose distribution in the cytosol is increased when the membrane is depolarized. This process is accompanied by an increase of fluorescence intensity. Thus, if the dye enters the cells upon depolarization of the resting membrane potential of the cells, for example, due to the blocking of voltage-dependent potassium channels, an increase of the fluorescence activity can be detected. In addition, the quantum yield of this oxonol derivative is advantageously favored by the decreased temperatures. Thus, the accompanying increase of signal intensity can cause a still improved signal-to-noise ratio, in addition to the above described inhibition of the cellular pump systems.

Another advantage relates to the stability of the signal to be read out. When the test is performed at 37 °C or at room temperature, the fluorescence signals (e.g., $Dibac_4(3)$ fluorescence) initially caused by the addition of a channel blocker (such as a potassium channel blocker) to cells can be restored to the initial condition after a short transient increase, inter alia, due to the activity of endogenous ion pumps. This means that the time slot for measuring an effect of substances on the ion channels is narrow, so that an on-line measurement is to be made within a very short time slot (typically less than 2 min) after the addition of the test substance, which puts high demands on the measuring device. If the test is performed at decreased temperatures, such a drop of the initially caused signal is not observed, or only so to a much reduced extent. This has the advantage that the measurement of an effect of a substance on an ion channel to be examined can be made also after several hours of incubation. This in turn significantly facilitates the screening of a large number of substances and thus increases the through-put. Many ion channels become accessible to screening only due to a conversion of a transient signal into a stable read-out parameter.

Apart from the above mentioned $Dibac_4(3)$ dye, other dyes of the bis-barbituric acid oxonol type may be used such as $DiSBAC_2(3)$ or $DiBAC_4(5)$ which are commercially available (e.g. by the supplier Molecular Probes). Also other oxonol dyes such as bis-isoxazolone oxonol dyes (e. g. Oxonol V and Oxonol VI) may be applied. Further voltage-sensitive indicators include carbocyanine derivatives (e.g. indo-, thia-, and oxa-carbocyanines as well as iodide derivatives of carbocyanines), rhodamine dyes, merocyanine 540 and styryl dyes. Among the styryl dyes, one might apply dyes of the aminonaphtylethenylpyridinium type such as di-4-ANEPPS, di-8-ANEPPS, di-2-ANEPEQ, di-8-ANEPPQ, di-12-ANEPPQ or di-1-ANEPIA which are all commercially available (Molecular Probes). Also RH-dyes of this or other suppliers may be used such as RH 414, RH 421, RH 795 or RH 237. As ion-sensitive indicators one might use well-known and commercially available calcium indicators (e.g. fluo-calcium indicators, fura indicators such as benzofuranyl derivatives, indo indicators such as indol derivatives, Calcium Green™ such as CAS No 186501-28-0 or Oregon Green™ such as CAS No 172646-19-4; Molecular Probes) or sodium/potassium indicators (e.g. SBFI, PBFI, Sodium Green $Na^+$ indicator, CoroNa Green $Na^+$ indicator, CoroNa Red $Na^+$ indicator; Molecular Probes). This and other suppliers also provide chelating agents for the conductance of ion channel experiments. Further, one might also apply the FLIPR Membrane Potential Assay Kit (Molecular Devices) used in the experiments set forth below.

In the following, the present invention is illustrated in various experimental examples.

## Example 1: HERG channel

CHO cells stably transfected with the voltage-dependent potassium channel HERG were trypsinized and centrifuged off. Thereafter, the cells were taken up in 1 x buffer (10 mM HEPES, pH 7.3, 140 mM $Na^+$, 2 mM $K^+$, 1 mM $MgCl_2$, 2 mM $CaCl_2$) with 4 μM $DiBAC_4(3)$ (Molecular Probes) and added at $2 \cdot 10^4$/well in 50 μl to a 384 well microtitration plate having a transparent bottom to which the following substances had been preliminarily added: 5 μl of buffer (2 mM $K^+$), 5 μl of buffer + 300 mM $K^+$, and 5 μl of buffer + 10 μM E4031/2 mM $K^+$.

$Dibac_4(3)$ served as a voltage-dependent fluorescence dye which enters the cell through the cell membrane upon depolarization of the cell membrane (e.g., caused by increasing the extracellular potassium concentration or by blocking potassium channels), where it binds to intracellular proteins and membranes, which results in an increase of fluorescence.

In the above described wells, the potassium concentration was brought to 2 mM (zero check) and 30 mM (control depolarization) by adding a suitable stock solution. As an antagonist of the HERG potassium channel, E4031 was added at a potassium concentration of 2 mM.

After 150 minutes of incubation on ice and subsequent incubation for 30 minutes at room temperature, the read-out was performed on a commercially available fluorescence reader (Fluostar, bmg) at an excitation wavelength of 485 nm and an emission wavelength of 525 nm.

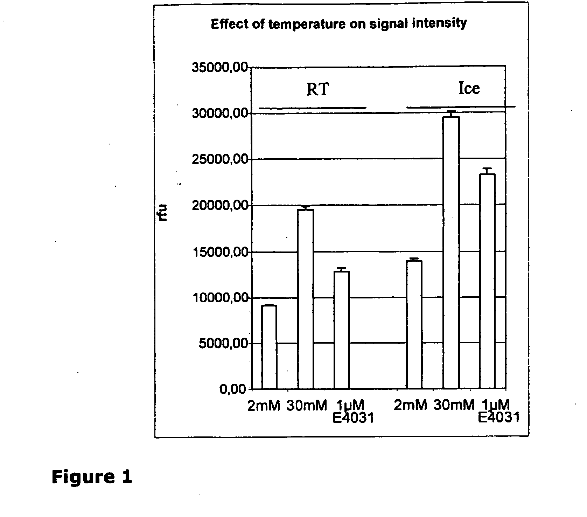

The results of the above described test method are summarized in Table 1 below and in Figure 1. It can be seen that the increase in fluorescence signal caused by blocking the HERG potassium channel by the antagonist E4031 is significantly stronger for incubation on ice as compared to room temperature. The enhancement of the signal increases from 41.76% at room temperature to 66.62% on ice.

**Table 1**

| 150 min of incubation on ice plus 30 min of subsequent incubation at room temperature | | | | | |
|---|---|---|---|---|---|
| | | Mean value of rfu* | Std. dev.** | Error [%] | Signal enhancement [%]*** |
| | 2 mM K$^+$ | 9037,67 | 211,27 | 2,34 | |
| | 30 mM K$^+$ | 19496,33 | 376,26 | 1,93 | 115,72 |
| | 1 µM E4031 | 12811,67 | 350,54 | 2,74 | 41,76 |
| 150 min of incubation on ice | | | | | |
| | 2 mM K$^+$ | 13997,67 | 217,94 | 1,56 | |
| | 30 mM K$^+$ | 29495,67 | 648,56 | 2,20 | 110,72 |
| | 1 µM E4031 | 23323,00 | 567,56 | 2,43 | 66,62 |
| * rfu: relative fluorescence intensity<br>** Std. dev.: standard deviation<br>*** Enhancement of the established fluorescence signal as compared to the zero check (fluorescence signal at 2 mM K$^+$) | | | | | |

In addition to the example 1 set forth above, further experiments were conducted to show the generic applicability of the method according to the present invention to other ion channels than the HERG-channel. Experiments were carried out utilizing the following ion channels: Kv1.1 (example 2), Kv1.5 (example 3), KCNQ1/KCNE1 (examples 4 and 6), Kv1.3 (examples 5 and 7) and SCN5a (example 8). The following general' description of chemicals, cell culture, membrane potential assays and data analysis relates to all of these additional examples 2-8.

### Chemicals

Chemicals were purchased from Sigma, Merck and Calbiochem. DiBAC4(3) was from Molecular Probes. FLIPR Membrane Potential Assay Kit (FMP) was from Molecular Devices. Toxins were purchased from Alomone Labs.

### Cell culture

CHO cell lines and HEK293 cell lines (wild type and stably transfected with the respective ion channels) were maintained and established at Evotec OAI AG (Hamburg, Germany). CHO cell lines were grown in 75 cm$^2$-flasks (Falcon) in 12 ml MEM ALPHA Medium (Gibco Invitrogen). HEK293 cell lines were grown in DMEM (Gibco Invitrogen). Both media were supplemented with 10% (v/v) fetal calf serum, 1% (v/v) L-glutamine solution (Gibco Invitrogen) and G-418 (geneticine) (800 µg/ml) and grown at 37°C and 5% CO$_2$. Cells were split according to standard cell culture protocols.
For performance of the membrane potential assay described in more detail below, the cells were either seeded (50 µl/well) into 384-well microplates (Falcon, Becton Dickinson), incubated for overnight at 37°C and 5% CO$_2$ in the above described media before subjecting them to the assay or directly seeded onto the plates at a density of 2·10$^4$ cells/well in assay buffer (see below).

### Membrane potential assay

In the present examples, this fluorescence-based assay makes use of fluorescent dyes (DiBAC4(3) and FMP dye) which either move into or out of the cells depending on the cells' membrane potential. Upon depolarisation of the cells, the dyes enter the cell and bind to intracellular hydrophobic sites, which in turn lead to an increased fluorescence intensity of the dyes.
The culture medium was removed from the cells grown in the microtiter plates. The cells were subsequently covered with 10 µl HEPES buffer (10 mM HEPES, pH 7.2, 5mM K$^+$, 140mM Na$^+$, 5mM Glucose, 1mM MgCl$_2$, 2mM CaCl$_2$). DiBAC4(3) was used at a 4µM concentration and FMP was used close to the manufacturer's instructions, respectively. Compounds to be tested in the assay were dissolved in DMSO at appropriate stock concentrations. Toxins were dissolved in PBS (phosphate-buffered saline buffer: 137mM NaCl, 2.7mM KCl, 4.3mM Na$_2$HPO$_4$·7H$_2$O, 1.4mM KH$_2$PO$_4$; pH~7.3) supplemented with 1mg/ml bovine serum albumin.
When adherent cells were used, the medium was removed and replaced by the respective assay buffer. The compounds or toxins were added subsequently. In case suspension cells were used, the compounds and toxins were added to the plate and the cells that were re-suspended in assay buffer after trypsinization were added into the wells.

The 30mM potassium added to the cells (see below) serves as a depolarization-positive control in all experiments.
DiBAC4(3) fluorescence signals were measured after the indicated time of incubation (mostly after several hours - see below) and temperature of incubation (in between 0°C and 4°C) using a BMG FLUOstar fluorescence reader (BMG Labtechnologies) or Safire reader (Tecan): excitation wavelength 485nm (12nm bandwidth), emission wavelength 520nm (35nm bandwidth) in case of DiBAC4(3) or the Safire-reader with excitation wavelength of 540nm (2.5nm bandwidth) and emission wavelength of 555 nm (2.5nm bandwidth) in case of FMP. Fluorescence was measured from below.

### Data analysis

The relative changes in fluorescence intensity were calculated as follows:

$$[\%]\ \text{increase}\ =\ 100\ \times\ \frac{(F_c - F_0)}{F_0}$$

$F_0$: fluorescence intensity [rfu] of CHO- or HEK293-cells expressing an ion channel under standard conditions (zero control, 2mM or 5mM potassium)

$F_c$: fluorescence intensity [rfu] of CHO- or HEK293-ion channel expressing cells in the presence of compounds / toxins

### Example 2: Kv 1.1

The following Table 2 shows the results obtained after incubation of the cells for 95 minutes at 4°C in the presence of 5 mM potassium (zero control), 30 mM potassium (positive control), and 100 nM of the toxins delta-DTX and DTX-K (both purchased from Alomone Labs, Israel):

**Table 2**

|  | [rfu] | Std.dev. | Error [%] | [%] increase |
|---|---|---|---|---|
| HEK1.1; 30mM K$^+$ | 31570.13 | 221.10 | 0.70 | 40.94 |
| HEK1.1; 5mM K$^+$ | 22399.53 | 414.47 | 1.85 | |
| HEK1.1; deltaDTx | 30732.47 | 233.29 | 0.76 | 37.20 |
| HEK1.1; DTx-K | 31707.80 | 861.33 | 2.72 | 41.56 |

"Rfu" denotes the relative fluorescence intensity.
"Sdt. dev." denotes the standard deviation.
"[%] increase" denotes the increase of the fluorescence signal of the cells in the presence of the respective ion channel blockers compared to the fluorescence signal of the cells under control conditions (see formula above)

The following Table 3 shows the results obtained after incubation of the same plate cells for 95 minutes at 4°C followed by an additional incubation for 35 minutes at 37°C in the presence of 5 mM potassium (zero control), 30 mM potassium (positive control), and 100 nM of the toxins delta-DTX and DTX-K (both purchased from Alomone Labs, Israel).

**Table 3**

|  | [rfu] | Sdt.dev. | Error [%] | [%] increase |
|---|---|---|---|---|
| HEK1.1; 30mM K$^+$ | 21754.33 | 117.32 | 0.54 | 18.73 |
| HEK1.1; 5mM K$^+$ | 18322.87 | 371.40 | 2.03 | |
| HEK1.1; deltaDTx | 20750.80 | 109.49 | 0.53 | 13.25 |
| HEK1.1; DTx-K | 21109.80 | 450.98 | 2.14 | 15.21 |

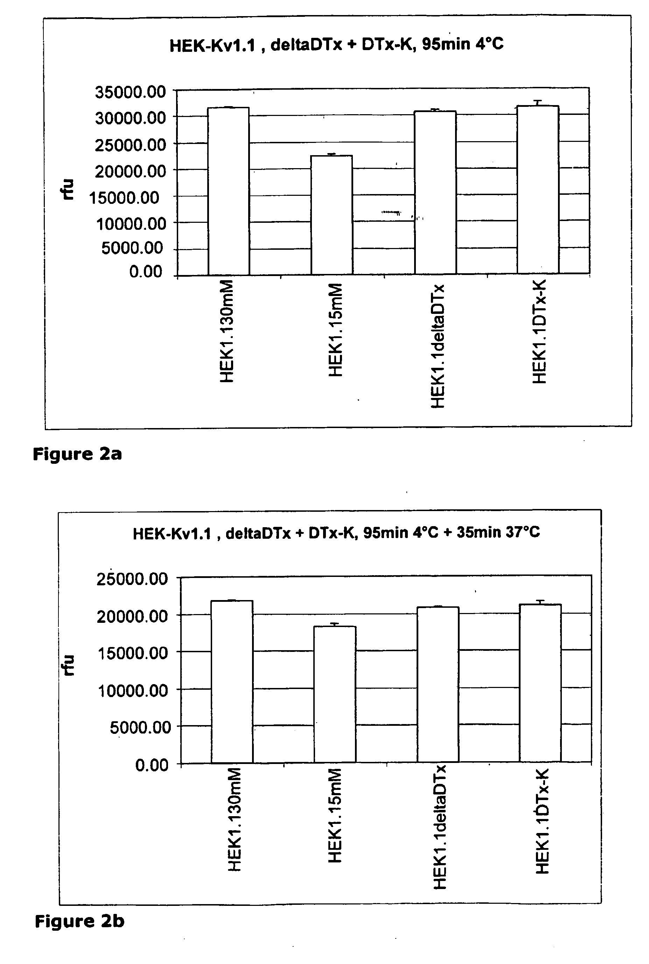

Incubating the cells in the presence of the inhibitors leads to a significantly more pronounced signal when the cells are incubated at low temperatures as taught by the present invention (compare [%] increase in case of the toxins deltaDTx and DTX-K: increase from approx. 14 % at 37°C to ~40 % at 4°C). See also Figures 2a and 2b.

**Example 3: Kv 1.5**

Semliki Forest Viruses carrying the Kv1.5 potassium channel were prepared according to the procedure described in Lundstrom et al., 1994, Eur. J. Biochem. 224:917-921 and were stored at -20 °C. Before use, the virus was activated with alpha-Chymotrypsinogen (0.2 mg/ml) for 15 min at room temperature. Activation of virus was stopped by addition of 1/50 volume aprotinin (20 mg/ml). CHO-cells were seeded the day before in 25cm$^2$-flasks, medium (see above under Cell Culture) was removed and replaced with 1ml fresh medium and 450 µl of the virus-containing supernatant as well as 20µl HEPES-buffer (10mM N-[2-Hydroxyethyl] piperazine-N'-[2-ethanesulfonic acid] pH 6.9). After incubation for 90 minutes another 5ml fresh medium was added and cells were incubated for 12 hours.

The following days, the cells were harvested by trypsinization, washed once in PBS (phosphate-buffered saline buffer: 137mM NaCl, 2.7mM KCl, 4.3mM $Na_2HPO_4 \cdot 7H_2O$, 1.4mM $KH_2PO_4$; pH~7.3) and re-suspended in assay buffer (10 mM HEPES, pH 7.2, 5mM K$^+$, 140mM Na$^+$, 5mM Glucose, 1mM $MgCl_2$, 2mM $CaCl_2$) containing FMP before adding to 384-wells loaded with a standard Kv1.5 blocker. Plates were incubated for 15 minutes at room temperature and subsequently cooled down to 1°C for 30 minutes. As can be seen in Table 4, the relative increase of the relative fluorescence units of the cells in the presence of the Kv1.5-specific blocker is significantly higher when cells are incubated at low temperature as taught by the present invention (63% relative increase compared to 26%).

**Table 4**

|  | [rfu] | Std.dev. | Error [%] | Increase [%] |
|---|---|---|---|---|
| **15 min at room temperature** |  |  |  |  |
| Zero control | 28275.86 | 718.22 | 2.54 |  |
| 30mM K$^+$ | 34496.79 | 1011.96 | 2.93 | 22.00 |
| 10µM compound | 35711.50 | 1849.73 | 5.18 | **26.30** |
| **30 min 1°C** |  |  |  |  |
| Zero control | 29256.36 | 1412.06 | 4.83 |  |
| 30mM K$^+$ | 47019.86 | 1579.57 | 3.36 | 37.78 |
| 10µM compound | 47737.07 | 2159.66 | 4.52 | **63.17** |

**Example 4: KCNQ1/KCNE1**

The following Table 5 shows the results obtained after incubation of the cells for 15 minutes at room temperature and additional 180 minutes in the presence of 2mM potassium (zero control), 30mM potassium (positive control), and 10µM of a standard antagonist according to the procedure described above using suspension cells and the FMP-dye. Again, the relative signal increase is significantly higher when cells are incubated at low temperature as taught by the present invention (109% relative increase compared to 24% increase when incubated at room temperature).

**Table 5**

|  | [rfu] | Std.dev. | Error [%] | Increase [%] |
|---|---|---|---|---|
| **30 min room temperature** |  |  |  |  |
| Zero control | 6037.80 | 370.07 | 6.13 |  |
| 30mM K$^+$ | 12067.03 | 752.27 | 6.23 | 99.86 |
| 10µM compound | 7517.19 | 359.33 | 4.78 | **24.50** |
| **180 min 1°C** |  |  |  |  |
| Zero control | 9968.25 | 749.07 | 7.51 |  |
| 30mM K$^+$ | 25157.61 | 1237.40 | 4.92 | 152.38 |
| 10µM compound | 20893.36 | 864.68 | 4.14 | **109.60** |

### Example 5: Kv 1.3

The following **Table 6** shows the results obtained after incubation of the cells for 10 minutes at room temperature and additional 110 minutes at 1°C in the presence of 5mM potassium (zero control), 30mM potassium (positive control), and 100 nM of Margatoxin (Alomone labs) according to the procedure described above using suspension cells and the FMP-dye. The relative signal is significantly higher when cells are incubated at low temperature as taught by the present invention (68% relative increase compared to 3% increase when incubated for 10 minutes at room temperature).

**Table 6**

|  | [rfu] | Std.dev. | Error [%] | Increase [%] |
|---|---|---|---|---|
| **10 min room temperature** |  |  |  |  |
| Zero control | 16994.63 | 491.21 | 2.89 |  |
| 30mM K$^+$ | 27062.25 | 460.50 | 1.70 | 23.91 |
| 100nM Margatoxin | 22479.50 | 968.56 | 4.31 | **2.93** |
| **110 min 1°C** |  |  |  |  |
| Zero control | 28251.75 | 1523.77 | 5.39 |  |
| 30mM K$^+$ | 60872.63 | 3569.17 | 5.86 | 115.46 |
| 100nM Margatoxin | 47474.00 | 2678.98 | 5.64 | **68.04** |

### Example 6: Screening application on KCNQ1/KCNE1

The examination of ion channels according to the present invention provides for an increased signal-to-noise ratio compared to methods known in the art.

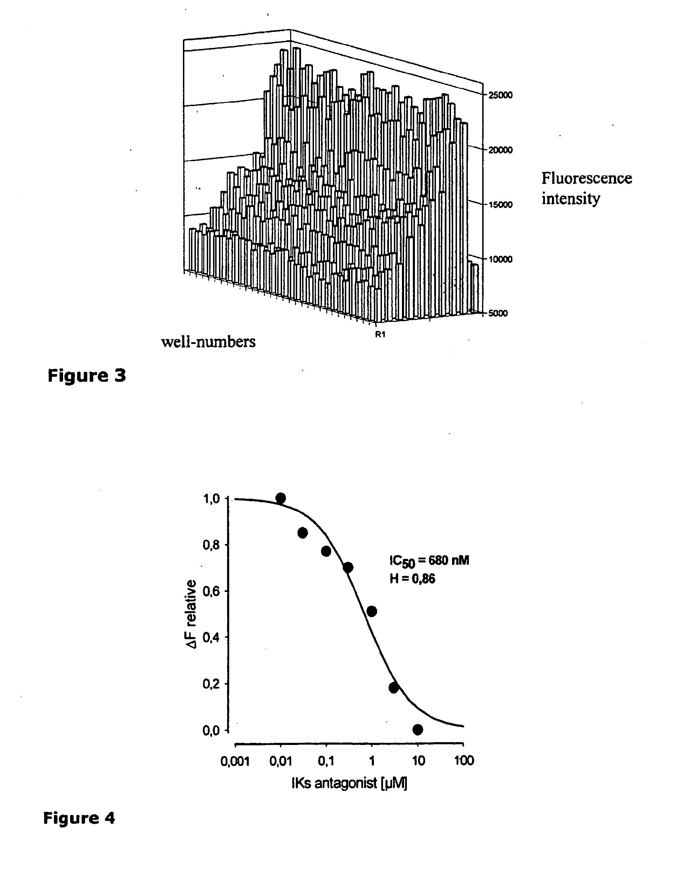

This results in statistical data sufficient for high-throughput screening campaigns. Investigations of control compounds on the KCNQ1/KCNE1 potassium channel (Iks) under HTS-conditions in 1536-well plates resulted in z'-factors of >0,6. Examples of the investigations of an Iks antagonist in 1536-well plates applying the present invention are depicted in figures 3 and 4. Figure 3 reflects the increase of fluorescence with increasing concentrations of the antagonist. In the first two and the-last two rows of the figure, the control values are shown (without compound), The fluorescence intensity in these control rows is significantly lower than the one in rows showing the fluorescence intensity of wells to which compounds have been applied. From the third column, results of increasing concentrations from 0.01 to 10 µM of the compound are shown. With increasing concentrations of the compound, the fluorescence intensity increases. An example for a respective concentration/response relation is shown in figure 4.

### Example 7: Screening application on Kv 1.3

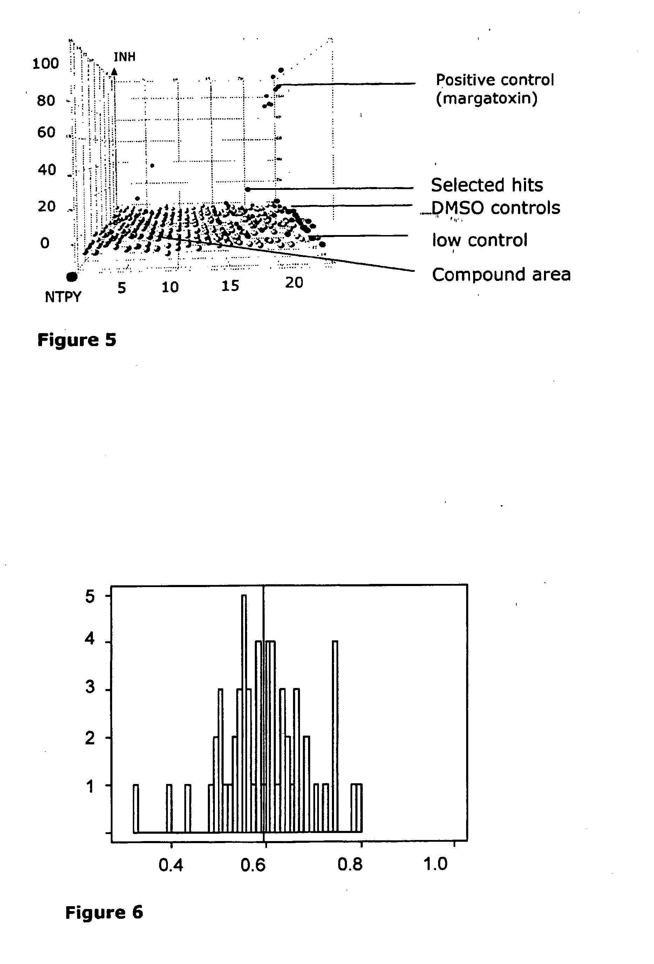

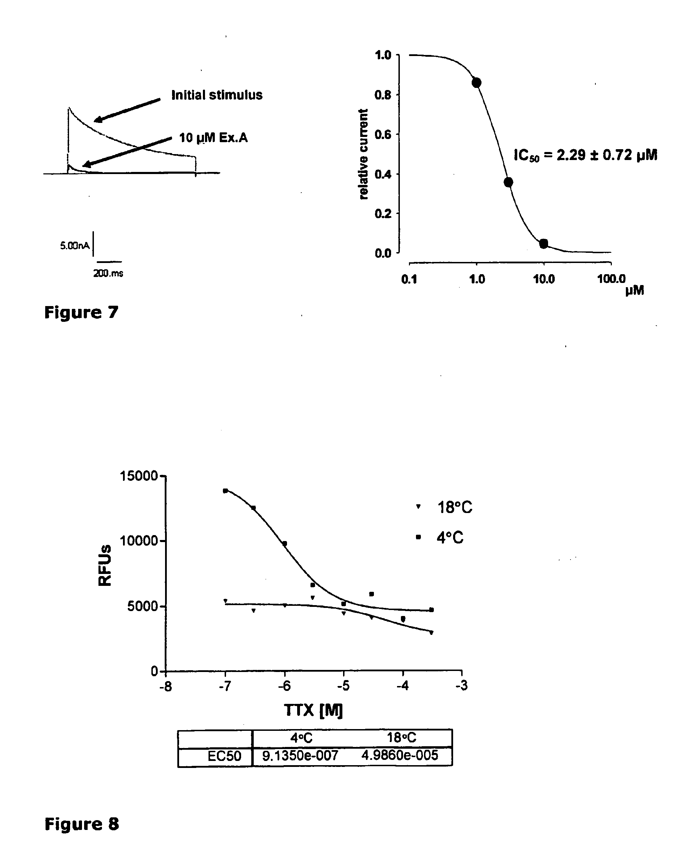

The invention was used for screens of larger numbers of compounds. In the present example, the measurements of 25.000 compounds on a Kv1.3 potassium channel were performed in 384-well plates. The investigations of the compounds resulted in an increase of the fluorescence when antagonists of the Kv1.3 channel were applied to the cells. Investigations of compounds expressing the Kv1.3 potassium channel are depicted in figure 5 (margenta: positive control margatoxin; blue: low controls; green: DMSO negative controls; grey: compound area; red: selected hits). The calculation of the mean z' of the investigations of 25.000 compounds on the Kv1.3 resulted in a value of approximately 0,6., as shown in figure 6. Compounds detected as hits by applying the method according to the present invention, were confirmed as Kv1.3 antagonists using electrophysiological methods (patch-clamp technique) as shown in figure 7.

### Example 8: SCN5a

In the present example, the sodium channel SCN5a expressed in a mammalian cell line is investigated. Cells were loaded in standard assay buffer using the Molecular Devices kit described above at room temperature for 30 mins. After loading, tetrodotoxin (TTX) was applied to different concentrations and incubated for a further 10 minutes after which Veratradine to an end concentration of 50µM was added and the plates were incubated on ice or at room temperature for various periods of time.

The following table shows the results after 17 minutes incubation.

**Table 7**

| TTX [μM] | 4°C [RFUs] | 18°C [RFUs] |
|----------|-----------|-------------|
| 3.00E+02 | 4687,0 | 2882,0 |
| 1.00E+02 | 4019,0 | 3816,0 |
| 3.00E+01 | 5873,0 | 4067,0 |
| 1.00E+01 | 5143,0 | 4401,0 |
| 3.00E+00 | 6610,0 | 5620,0 |
| 1.00E+00 | 9772,0 | 5034,0 |
| 3.00E-01 | 12512,0 | 4636,0 |
| 1.00E-01 | 13840,0 | 5379,0 |

The IC50 value was calculated for both incubation conditions and is shown in figure 8. Incubation of cells with compounds at low temperature according to the present invention leads to much improved sensitivity of detection as well as signal-to-noise ratio. At low temperature incubation, the reported IC 50 value is in excellent agreement with literature.

**Claims**

1. A method for minimising the risk that the potential value measured is biased due to counter-regulation mechanisms when examining the activity of ion channels in the research on pharmaceutically active substances involving the medium- or high-throughput screening of potentially or established active pharmaceutical substances, comprising the following steps:

    - providing a sample comprising membrane-bound ion channels; and
    - determining as an indicator of the activity of the ion channels a value of a measuring parameter chosen from a membrane potential of a cell, cell organelle or vesicle, a measure of said membrane potential, an extracellular, intracellular, extravesicular and/or intravesicular ion concentration or a measure thereof before, during and/or after the addition of a test substance which potentially influences the activity of the ion channels;

    wherein said determining of the value of the measuring parameter is performed by fluorescence methods at a temperature of ≤ 10°C to minimize the risk that the potential value measured is biased due to counter-regulation mechanisms.

2. The method according to claim 1, **characterized in that** said determining of the value of the measuring parameter is performed at a temperature of ≤ 5 °C, especially ≤ 2 °C.

3. The method according to claim 1 or 2, **characterized in that** said determining of the value of the measuring parameter is performed at a temperature of from 10 °C to -4 °C, especially from 5 °C to -4 °C, more preferably from 5 °C to 0 °C, even more preferably from 2 °C to 0 °C.

4. The method according to any of the preceding claims, **characterized in that** the sample comprises one or more cells or cell organelles which have ion channels, in particular human or animal cells or cell organelles.

5. The method according to any of the preceding claims, **characterized in that** the sample comprises one or more vesicles which have ion channels.

6. The method according to any of the preceding claims, **characterized in that** the membrane-bound ion channels are ion channels embedded into a membrane of cells, cell organelles, vesicles or embedded into an artificial membrane.

7. The method according to any of the preceding claims, **characterized in that** the activity of a transmitter-dependent ion channel is examined.

8. The method according to any of the preceding claims, **characterized in that** the activity of a voltage-sensitive ion channel is examined.

9. The method according to any of the preceding claims, **characterized in that** the activity of a potassium channel, chloride channel, sodium channel or calcium channel is examined.

10. The method according to any of the preceding claims **characterized in that** an optical response of (i) a carbocyanine derivative, in particular a thia-,indo-, or oxa-carbocyanine or an iodide derivative of a carbocyanine, (ii) a rhodamine dye, (iii) an oxonol dye, (iv) merocyahlne 540, or (v) a styryl dye serves as a measure of the membrane potential.

11. The method according to any of the preceding claims, **characterized in that** the fluorescence emission of a voltage-sensitive fluorescent dye, preferably a DiBAC dye, more preferably the dye $Dibac_4(3)$, serves as a measure of the membrane potential.

12. The method according to any of the preceding claims, **characterized in that** the ion concentration, especially the ion concentration of calcium, is measured by means of chelating agents.

13. The method according to any of the preceding claims, **characterized in that** the values of several measuring parameters are determined.

14. The method according to any of the preceding claims for use in the identification of potentially active pharmaceutical substances or the determination of side effects of potentially or established active pharmaceutical substances.

15. The method according to any of the preceding claims wherein as an ion-sensitive indicator a calcium indicator, in particular a fluo-calcium indicator, a fura indicator, an indo indicator, Calcium Green™, or Oregon Green™ is used.

16. The method according to any of the preceding claims wherein as an ion-sensitive indicator a sodium or potassium indicator, preferably a fluorescent sodium or potassiumindicator, in particular SBFI, PBFI, Sodium Green $Na^+$ indicator, CoroNa Green $Na^+$ indicator, or CoroNa Red $Na^+$ indicator is used.

17. The method according to any of the preceding claims wherein as an oxonol dye a bis-isoxazolone oxonol dye or a bis-barbituric acid oxonol (DiBAC) dye, in particular $DiBAC_4(3)$, $DiSBAC_2(3)$ or $DiBAC_4(5)$ is used.

18. The method according to any of the preceding claims wherein as an styryl dye an ANEP (AminoNaphthylEthehylPyridinium) dye, in particular di-4-ANEPPS, di-8-ANEPPS, di-2-ANEPEQ, di-8-ANEPPQ, di-12-ANEPPQ, di-1-ANEPIA, or a-dialkylaminophenylpolyenylpyridinium dye (RH dye), in particular RH 414, RH 421, RH 795 or RH 237 is used.

19. Use of a flow cytometer, a fluorescence microscope or fluorescence plate reader for the conductance of the method according to any of claims 1 to 18.

**Patentansprüche**

1. Verfahren zum Minimieren des Risikos, dass der potentielle Messwert einem systematischen Fehler aufgrund von Gegenregulationsmechanismen unterliegt, wenn man bei der Erforschung von pharmazeutischen Wirkstoffen die Aktivität von Ionenkanälen untersucht, wobei ein Mittel-oder Hochdurchsatz-Screening von potentiellen oder etablierten pharmazeutischen Wirkstoffen beteiligt ist, umfassend die folgenden Schritte:

- Bereitstellen einer Probe, die membrangebundene Ionenkanäle umfasst; und
- Bestimmen eines Werts eines Messparameters, der aus einem Membranpotential einer Zelle, eines Zellorganells oder Vesikels, einem Maß für das Membranpotential, einer extrazellulären, intrazellulären, extravesikulären und/oder intravesikulären Ionenkonzentration oder einem Maß dafür ausgewählt ist, als Indikator für die Aktivität der Ionenkanäle vor, während und/oder nach der Zugabe einer Testsubstanz, die die Aktivität der Ionenkanäle potentiell beeinflusst;

wobei die Bestimmung des Werts des Messparameters durch Fluoreszenzverfahren bei einer Temperatur von ≤ 10 °C durchgeführt wird, so dass das Risiko, dass der potentielle Messwert einem systematischen Fehler aufgrund von Gegenregulationsmechanismen unterliegt, minimiert wird.

**2.** Verfahren gemäß Anspruch 1, **dadurch gekennzeichnet, dass** die Bestimmung des Werts des Messparameters bei einer Temperatur von ≤ 5 °C, insbesondere ≤ 2 °C, durchgeführt wird.

**3.** Verfahren gemäß Anspruch 1 oder 2, **dadurch gekennzeichnet, dass** die Bestimmung des Werts des Messparameters bei einer Temperatur von 10 °C bis -4 °C, insbesondere 5 °C bis -4 °C, besonders bevorzugt 5 °C bis 0 °C, ganz besonders bevorzugt 2 °C bis 0 °C, durchgeführt wird.

**4.** Verfahren gemäß einem der vorstehenden Ansprüche, **dadurch gekennzeichnet, dass** die Probe eine oder mehrere Zellen oder Zellorganellen, die Ionenkanäle aufweisen, insbesondere menschliche oder tierische Zellen oder Zellorganellen, umfasst.

**5.** Verfahren gemäß einem der vorstehenden Ansprüche, **dadurch gekennzeichnet, dass** die Probe ein oder mehrere Vesikel, die Ionenkanäle aufweisen, umfasst.

**6.** Verfahren gemäß einem der vorstehenden Ansprüche, **dadurch gekennzeichnet, dass** es sich bei den membrangebundenen Ionenkanälen um Ionenkanäle handelt, die in eine Membran von Zellen, Zellorganellen, Vesikeln oder in eine künstliche Membran eingebettet sind.

**7.** Verfahren gemäß einem der vorstehenden Ansprüche, **dadurch gekennzeichnet, dass** die Aktivität eines transmitterabhängigen Ionenkanals untersucht wird.

**8.** Verfahren gemäß einem der vorstehenden Ansprüche, **dadurch gekennzeichnet, dass** die Aktivität eines spannungsabhängigen Ionenkanals untersucht wird.

**9.** Verfahren gemäß einem der vorstehenden Ansprüche, **dadurch gekennzeichnet, dass** die Aktivität eines Kaliumkanals, Chloridkanals, Natriumkanals oder Calciumkanals untersucht wird.

**10.** Verfahren gemäß einem der vorstehenden Ansprüche, **dadurch gekennzeichnet, dass** eine optische Reaktion von (i) einem Carbocyanin-Derivat, insbesondere einem Thia-, Indo- oder Oxacarbocyanin oder einem Iodderivat eines Carbocyanins, (ii) einem Rhodaminfarbstoff, (iii) einem Oxonolfarbstoff, (iv) Merocyanin 540 oder (v) einem Styrylfarbstoff als Maß für das Membranpotential dient.

**11.** Verfahren gemäß einem der vorstehenden Ansprüche, **dadurch gekennzeichnet, dass** die Fluoreszenzemission eines spannungsabhängigen Fluoreszenzfarbstoffs, vorzugsweise eines DiBAC-Farbstoffs, besonders bevorzugt des Farbstoffs $Dibac_4(3)$, als Maß für das Membranpotential dient.

**12.** Verfahren gemäß einem der vorstehenden Ansprüche, **dadurch gekennzeichnet, dass** die Ionenkonzentration, insbesondere die Ionenkonzentration von Calcium, mittels Chelatisierungsmitteln gemessen wird.

**13.** Verfahren gemäß einem der vorstehenden Ansprüche, **dadurch gekennzeichnet, dass** die Werte von mehreren Messparametern bestimmt werden.

**14.** Verfahren gemäß einem der vorstehenden Ansprüche zur Verwendung bei der Identifizierung von potentiellen pharmazeutischen Wirkstoffen oder bei der Bestimmung von Nebenwirkungen von potentiellen oder etablierten pharmazeutischen Wirkstoffen.

**15.** Verfahren gemäß einem der vorstehenden Ansprüche, wobei ein Calcium-Indikator, insbesondere ein fluoreszierender Calcium-Indikator, ein Fura-Indikator, ein Indo-Indikator, Calcium Green™ oder Oregon Green™ als ionenempfindlicher Indikator verwendet wird.

**16.** Verfahren gemäß einem der vorstehenden Ansprüche, wobei ein Natrium-oder Kalium-Indikator, vorzugsweise ein fluoreszierender Natrium- oder Kalium-Indikator, insbesondere SBFI, PBFI, Sodium-Green-$Na^+$-Indikator, CoroNa-Green-$Na^+$-Indikator oder CoroNa-Red-$Na^+$-Indikator als ionenempfindlicher Indikator verwendet wird.

**17.** Verfahren gemäß einem der vorstehenden Ansprüche, wobei ein Bisisoxazolon-Oxonolfarbstoff oder ein Bisbarbitursäure-Oxonoi(DiBAC)-Farbstoff, insbesondere DiBAC$_4$(3), DiSBAC$_2$(3) oder DiBAC$_4$(5), als Oxonolfarbstoff verwendet wird.

**18.** Verfahren gemäß einem der vorstehenden Ansprüche, wobei ein ANEP-(AminoNaphthylEthenylPyridinium)-Farbstoff, insbesondere Di-4-ANEPPS, Di-8-ANEPPS, Di-2-ANEPEQ, Di-8-ANEPPQ, Di-12-ANEPPQ, Di-1-ANEPIA, oder ein Dialkylaminophenylpolyenylpyridinium-Farbstoff (RH-Farbstoff), insbesondere RH 414, RH 421, RH 795 oder RH 237, als Styrylfarbstoff verwendet wird.

**19.** Verwendung eines Durchflusscytometers, eines Fluoreszenzmikroskops oder eines Fluoreszenzplattenablesegeräts zur Durchführung des Verfahren gemäß einem der Ansprüche 1 bis 18.

**Revendications**

**1.** Procédé pour minimiser le risque que la valeur de l'indice potentiel mesurée soit biaisée du fait de mécanismes de contre-régulation lors de l'examen de l'activité des canaux ioniques au cours de recherches sur des substances pharmaceutiquement actives impliquant le criblage à rendement moyen ou élevé de substances pharmaceutiques potentiellement ou avérées actives, comprenant les étapes suivantes consistant à :

- fournir un échantillon comprenant des canaux ioniques membranaires ; et
- déterminer, en tant qu'indicateur de l'activité des canaux ioniques, une valeur d'un paramètre de mesure choisi parmi un potentiel de membrane d'une cellule, d'une organite ou vésicule cellulaire, une mesure dudit potentiel de membrane, une concentration ionique extracellulaire, intracellulaire, extravésiculaire et/ou intravésiculaire ou une de ses mesures avant, pendant et/ou après l'addition d'une substance d'essai qui influe potentiellement sur l'activité des canaux ioniques ;

dans lequel ladite détermination de la valeur du paramètre de mesure est réalisée par des procédés de fluorescence à une température de 10°C afin de minimiser le risque que la valeur de potentiel mesurée soit biaisée du fait de mécanismes de contre-régulation.

**2.** Procédé selon la revendication 1, **caractérisé en ce que** ladite détermination de la valeur du paramètre de mesure est réalisée à une température ≤ 5°C, en particulier ≤ 2°C.

**3.** Procédé selon la revendication 1 ou 2, **caractérisé en ce que** ladite détermination de la valeur du paramètre de mesure est réalisée à une température de 10°C à -4°C, en particulier de 5°C à -4°C, de manière plus particulièrement préférée de 5°C à 0°C, de manière encore plus particulièrement préférée de 2°C à 0°C.

**4.** Procédé selon l'une quelconque des revendications précédentes, **caractérisé en ce que** l'échantillon comprend une ou plusieurs cellules ou organites cellulaires qui possèdent des canaux ioniques, en particulier des cellules humaines ou animales ou des organites cellulaires.

**5.** Procédé selon l'une quelconque des revendications précédentes, **caractérisé en ce que** l'échantillon comprend une ou plusieurs vésicules qui présentent des canaux ioniques.

**6.** Procédé selon l'une quelconque des revendications précédentes, **caractérisé en ce que** les canaux ioniques liés à la membrane sont des canaux ioniques enfouis dans une membrane de cellules, d'organites cellulaires, de vésicules ou enfouis dans une membrane artificielle.

**7.** Procédé selon l'une quelconque des revendications précédentes, **caractérisé en ce que** l'activité d'un canal ionique dépendant d'un émetteur est examinée.

**8.** Procédé selon l'une quelconque des revendications précédentes, **caractérisé en ce que** l'activité d'un canal ionique sensible aux variations de tension est examinée.

**9.** Procédé selon l'une quelconque des revendications précédentes, **caractérisé en ce que** l'activité d'un canal potassique, d'un canal de chlorure, d'un canal sodique ou d'un canal calcique est examinée.

**10.** Procédé selon l'une quelconque des revendications précédentes, **caractérisé en ce qu'**une réponse optique (i) d'un dérivé carbocyanine, en particulier une thia-, indo-ou oxa-carbocyanine ou un dérivé iodure d'une carbocyanine, (ii) d'un colorant rhodamine, (iii) d'un colorant oxonol, (iv) de la mérocyanine 540, ou (v) d'un colorant styryle, sert de mesure du potentiel de membrane.

**11.** Procédé selon l'une quelconque des revendications précédentes, **caractérisé en ce que** l'émission de fluorescence d'un colorant fluorescent sensible au variations de tension, de préférence un colorant DiBAC, de manière plus particulièrement préférée le colorant $Dibac_4(3)$, sert de mesure du potentiel de membrane.

**12.** Procédé selon l'une quelconque des revendications précédentes, **caractérisé en ce que** la concentration ionique, en particulier la concentration ionique en calcium, est mesurée au moyen d'agents de chélation.

**13.** Procédé selon l'une quelconque des revendications précédentes, **caractérisé en ce que** les valeurs de plusieurs paramètres de mesure sont déterminées.

**14.** Procédé selon l'une quelconque des revendications précédentes en vue d'une utilisation dans l'identification de substances pharmaceutiques potentiellement actives ou la détermination d'effets secondaires de substances pharmaceutiques potentiellement ou avérées actives.

**15.** Procédé selon l'une quelconque des revendications précédentes, dans lequel, en tant qu'indicateur sensible aux ions on utilise, un indicateur de calcium, en particulier un indicateur de Fluo-calcium, un indicateur Fura, un indicateur Indo, le Calcium Green™ ou l'Oregon Green™.

**16.** Procédé selon l'une quelconque des revendications précédentes, dans lequel, en tant qu'indicateur sensible aux ions, on utilise un indicateur de sodium ou de potassium, de préférence un indicateur de sodium ou de potassium fluorescent, en particulier l'indicateur SBFT, PBFI, Sodium Green $Na^+$, l'indicateur CoroNa Green $Na^+$ ou l'indicateur CoroNa Red $Na^+$.

**17.** Procédé selon l'une quelconque des revendications précédentes, dans lequel, en tant que colorant oxonol, on utilise un colorant oxonol bis-isoxazolone ou un colorant oxonol de l'acide bis-barbiturique (DiBAC), en particulier $DiBAC_4$ (3), $DiSBAC_2(3)$ ou $DiBAC_4(5)$.

**18.** Procédé selon l'une quelconque des revendications précédentes, dans lequel, en tant que colorant styryle, on utilise un colorant ANEP (AminoNaphtylÉthénylPyridinium), en particulier di-4-ANEPPS, di-8-ANEPPS, di-2-ANEPEQ, di-8-ANEPPQ, di-12-ANEPPQ, di-1-ANEPIA, ou un colorant dialkylaminophénylpolyénylpyridinium (colorant RH), en particulier RH 414, RH 421, RH 795 ou RH 237.

**19.** Utilisation d'un cytomètre en flux, d'un microscope à fluorescence ou d'un lecteur de plaques à fluorescence en vue de la réalisation du procédé selon l'une quelconque des revendications 1 à 18.

**Figure 1**

**HEK-Kv1.1 , deltaDTx + DTx-K, 95min 4°C**

Figure 2a

**HEK-Kv1.1 , deltaDTx + DTx-K, 95min 4°C + 35min 37°C**

Figure 2b

Fluorescence
intensity

well-numbers

**Figure 3**

IC$_{50}$ = 680 nM
H = 0,86

$\Delta$F relative

IKs antagonist [µM]

**Figure 4**

**Figure 5**

**Figure 6**

**Figure 7**

| | 4°C | 18°C |
|---|---|---|
| EC50 | 9.1350e-007 | 4.9860e-005 |

**Figure 8**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SEVCIK.** *Biochimica et Biophysica Acta,* 1983, vol. 728, 305-310

- **LUNDSTROM et al.** *Eur. J. Biochem.,* 1994, vol. 224, 917-921